# EUROPEAN PATENT APPLICATION

(11) **EP 0 866 045 A1**
(43) Date of publication of application: **23.09.1998**
(21) Application number: 98103786.4
(22) Date of filing: 04.03.1998
(51) Int. Cl.: C07C 2/02, C07C 15/40, B01J 23/04

(54) **Process for producing a monoalkenylaromatic hydrocarbon compound**

(30) Priority: 17.03.1997 JP 63144/97
(71) Applicant: MITSUBISHI GAS CHEMICAL COMPANY, INC., Chiyoda-ku, Tokyo (JP)
(72) Inventor: Fushimi, Norio, 22, Wadai, Tsukuba-shi, Ibaraki-ken (JP); Takagawa, Makoto, 22, Wadai, Tsukuba-shi, Ibaraki-ken (JP)
(74) Representative: Türk, Gille, Hrabal, Leifert

(57) **Abstract**

A solid material is obtained by calcining a mixture of a carbonate or a hydroxide of an alkaline earth metal and a potassium compound, adding an alkali metal to the obtained calcined product under an atmosphere of an inert gas, and subsequently heat treating the obtained mixture at a temperature which is the same as or higher than the melting point of the alkali metal. A process for producing a monoalkenylaromatic hydrocarbon which comprises alkenylating a side chain of an aromatic hydrocarbon compound having at least one hydrogen atom at the α-position of the side chain with a conjugated diene having 4 or 5 carbon atoms in the presence of a catalyst comprising the obtained solid material is disclosed.

In accordance with the above process, a monoalkenenyl aromatic hydrocarbon can be produced safely at a low cost, and the process has a remarkable industrial advantage.

## Description

### BACKGROUND OF THE INVENTION

### 1. Field of the Invention

The present invention relates to a process for producing a monoalkenylaromatic hydrocarbon compound by alkenylating a side chain of an aromatic hydrocarbon compound with a conjugated diene having 4 or 5 carbon atoms.

Monoalkenylaromatic hydrocarbon compounds, such as monoalkenylbenzenes, are useful as intermediate materials for various organic compounds, such as monomers of macromolecular compounds, drugs, and the like. For example, 5-(o-tolyl)-2-pentene produced from o-xylene and 1,3-butadiene can be converted into industrially useful 2,6-naphthalenedicarboxylic acid by the successive ring closure, dehydrogenation, isomerization, and oxidation.

### 2. Description of the Relates Arts

To produce a monoalkenylbenzene by alkenylating a side chain of an aromatic hydrocarbon compound with a conjugated diene having 4 or 5 carbon atoms, processes using an alkali metal, such as sodium and potassium, or an alloy thereof, as the catalyst have been known.

For example, a process using sodium metal as the catalyst is described in German Patent No. 557514. A process using sodium metal supported on an alkaline earth metal oxide is described in J. Org. Chem., Vol. 30 (1965), Pages 82 to 84.

A process using potassium metal as the catalyst is described in Japanese Patent Publication No. Showa 50(1975)-17973. Processes using a potassium/sodium alloy or a mixture of potassium and sodium metals are described in Japanese Patent Publication Nos. Showa 50(1975)-17975 and Showa 51(1976)-8930. Processes using potassium metal supported on an alkali metal oxide or an alkaline earth metal oxide are described in United States Patent No. 3,244,758 and the above reference (J. Org. Chem).

Processes using a mixture obtained by heat treatment of a potassium compound and sodium metal at a temperature of or above 300°C or 350°C as the catalyst are described in Japanese Patent Application Laid-Open Nos. Showa 47(1972)-27929 and Showa 47(1972)-31935.

In the conventional technologies described above, when sodium metal is used as the catalyst without any treatment or in the form supported on an alkaline earth metal oxide, the catalyst shows neither sufficient activity nor sufficient selectivity and cannot practically be used in an industrial process. A process using potassium metal, a potassium/sodium alloy, or a mixture of potassium and sodium metals as the catalyst shows high activity. However, the catalyst reacts violently with oxygen and water. Therefore, when the process is industrially conducted, possibility of hazard such as fire and explosion is large, and the process has many problems with respect to safety.

The process using a mixture obtained by heat treatment of sodium metal and a potassium compound at a high temperature as the catalyst is characterized in that neither potassium metal nor a potassium alloy is directly used for the reaction. However, the process is not always practical as an industrial process because the activity is not sufficient and powder which is easily ignited must be treated at a high temperature.

### SUMMARY OF THE INVENTION

An object of the present invention is to provide a process for producing a monoalkenylaromatic hydrocarbon compound by alkenylating a side chain of an aromatic hydrocarbon compound with a conjugated diene having 4 or 5 carbon atoms at a high yield and a low cost in a safe process.

As the result of extensive studies by the present inventors to solve the above problems on the process for producing a monoalkenylaromatic hydrocarbon compound by alkenylating the α-position of a side chain of an aromatic hydrocarbon compound with a conjugated diene having 4 or 5 carbon atoms, it was found that the monoalkenylaromatic hydrocarbon compound can be produced at a high yield and a low cost in a safe process by using a solid basic catalyst which is obtained by calcining a mixture of a carbonate or a hydroxide of an alkaline earth metal and a potassium compound, adding an alkali metal to the obtained calcined product under an atmosphere of an inert gas, and subsequently heat treating the obtained mixture at a temperature which is the same as or high than the melting point of the alkali metal. The present invention has been completed on the basis of this knowledge.

Accordingly, the present invention provides a process for producing a monoalkenylaromatic hydrocarbon which comprises alkenylating a side chain of an aromatic hydrocarbon compound having at least one hydrogen atom at the α-position of the side chain with a conjugated diene having 4 or 5 carbon atoms in the presence of a catalyst comprising a solid material which has been obtained by calcining a mixture of a carbonate or a hydroxide of an alkaline earth metal and a potassium compound, adding an alkali metal to the obtained calcined product under an atmosphere of an inert gas, and subsequently heat treating the obtained mixture at a temperature which is the same as or higher than the melting point of the alkali metal.

The catalyst used in the process of the present invention shows a remarkably high activity in the alkenylation reaction of a side chain of an aromatic hydrocarbon compound with conjugated dienes. The catalyst exhibits sufficient activity even when the catalyst is prepared at a relatively low temperature as long as the temperature of the preparation is the same as or higher than the melting temperature of the used alkali metal. Monoalkenylaromatic hydrocarbon compounds, such as monoalkenylbenzenes, can be obtained at a high yield and a high selectivity even when a small amount of the catalyst is used, and the catalyst can be handled easily.

### DESCRIPTION OF THE PREFERRED EMBODIMENTS

As the aromatic hydrocarbon compound having at least one hydrogen atom bonded to the α-position of a side chain which is used as a raw material in the present invention, a monocyclic aromatic hydrocarbon compound or a polycyclic aromatic hydrocarbon compound is used.

Examples of the monocyclic aromatic hydrocarbon compound include monoalkylbenzenes, such as toluene, ethylbenzene, n-propylbenzene, isopropylbenzene, n-butylbenzene, sec-butylbenzene, and isobutylbenzene; dialkylbenzenes, such as o-xylene, m-xylene, p-xylene, o-ethyltoluene, m-ethyltoluene, p-ethyltoluene, o-diethylbenzene, m-diethylbenzene, and p-diethylbenzene; trialkylbenzenes, such as mesitylene and pseudocumene; and polyalkylbenzenes (tetraalkylbenzenes, pentaalkylbenzenes, hexaalkylbenzenes, and the like), such as 1,2,3,5-tetramethylbenzene, 1,2,4,5-tetramethylbenzene, pentamethylbenzene, and hexamethylbenzene. Examples of the polycyclic aromatic hydrocarbon compound include monoalkylnaphthalenes, such as 1-methylnaphthalene and 2-methylnaphthalene; dialkylnaphthalenes, such as dimethylnaphthalenes; tetrahydronaphthalene; and indan.

As the conjugated diene having 4 or 5 carbon atoms which is used as another raw material, 1,3-butadiene, 1,3-pentadiene, or isoprene is used.

Examples of the carbonates and the hydroxides of alkaline earth metals used for the catalyst include magnesium carbonate, magnesium hydroxide, calcium carbonate, and calcium hydroxide. These compounds may be used singly or as a mixture of two or more compounds. Carbonates, such as magnesium carbonate and calcium carbonate, are preferably used in order to obtain a catalyst showing good dispersion and having good powder properties.

As the potassium compound used by mixing with the carbonate or the hydroxide of an alkaline earth metal, potassium hydroxide, potassium carbonate, or potassium hydrogencarbonate is used.

The catalyst used in the present invention is prepared by calcining a mixture of the above carbonate or hydroxide of an alkaline earth metal and the potassium compound, adding an alkali metal to the obtained calcined product, and subsequently heat treating the obtained mixture. As the alkali metal used in this preparation, lithium, sodium, potassium, rubidium, or cesium is used. Among these metals, sodium and potassium are preferably used.

The carbonate or the hydroxide of an alkaline earth metal and the potassium compound are used in such amounts that the amount of potassium metal is 0.001 to 5 parts by weight, preferably 0.002 to 2 parts by weight, per 1 part by weight of the carbonate or the hydroxide of an alkaline earth metal. When the amount of the potassium compound is less than this range, there are drawbacks in that side reactions, such as isomerization of the produced monoalkenylaromatic compound such as monoalkenylbenzene, tends to take place, and that decrease in the catalyst activity is fast. Moreover, a large amount of the catalyst is necessary for maintaining a high activity of the catalyst to cause complicated treatments after the reaction. When the amount of the potassium compound is more than the above range, the potassium compound occupies the majority of the mixture. Therefore, the effect of the carbonate or the hydroxide of an alkaline earth metal is exhibited almost negligibly, and a catalyst which shows a high activity and can be handled easily is not prepared. Therefore, such amounts are not preferable.

To mix the carbonate or the hydroxide of an alkaline earth metal and the potassium compound, any of dry processes and wet processes may be used as long as both components are sufficiently mixed and dispersed to each other. For the purpose of mixing the components uniformly, it is preferable that the mixing is conducted in accordance with a wet process. When the mixing is conducted in accordance with a dry process, it is preferable that the carbonate or the hydroxide of an alkaline earth metal and the potassium compound are mixed and dispersed by heating to a temperature which is the same as or higher than the melting point of the used potassium compound. When the mixing is conducted in accordance with a wet process, for example, the carbonate or the hydroxide of an alkaline earth metal is added to an aqueous solution of the potassium compound and the obtained mixture is dried after being stirred and mixed well.

The mixture obtained by the above process is calcined and then mixed with the alkali metal. The temperature of calcination is 250 to 700°C, preferably 350 to 600°C.

The catalyst used in the present invention is obtained by mixing the alkali metal with the product obtained by calcining the mixture of the carbonate or the hydroxide of an alkaline earth metal and the potassium compound in an atmosphere of an inert gas and heat treating the obtained mixture.

The amount of the alkali metal mixed above is in the range of 0.01 to 10 atoms, preferably in the range of 0.02 to 5 atoms, of the alkali metal per 1 atom of the potassium atom in the potassium compound. When the amount is outside the range, the effects of the alkali metal and the potassium compound are not sufficiently exhibited, and a large amount of the catalyst must be used in order to obtain the necessary catalyst activity.

The preparation of the catalyst by mixing the alkali metal with the compound obtained by calcining the mixture of the carbonate or the hydroxide of an alkaline earth metal and the potassium compound is conducted by heating and mixing the components in an atmosphere of an inert gas at a temperature which is the same as or higher than the melting point of the alkali metal. The inert gas is a gas which is substantially inert to the prepared catalyst under the condition of the catalyst preparation. Specific examples of the inert gas include nitrogen, helium, and argon. When lithium is used as the alkali metal, nitrogen is not preferable because nitrogen reacts with lithium.

The temperature of the heat treatment is in the range of the melting point of the alkali metal to 500°C, preferably in the range of the melting point of the alkali metal to 300°C. The time of the heat treatment is generally in the range of 5 to 300 minutes. When the temperature of the heat treatment is lower than the melting temperature of the alkali metal, the alkali metal does not melt, and it is difficult that effective contact between the components through uniform mixing of the components is achieved when the alkali metal is mixed with the compound prepared from the carbonate or the hydroxide of an alkaline earth metal and the potassium compound. Therefore, the preparation of the catalyst takes time, and such a temperature is not practical. The catalyst can be prepared at a temperature exceeding 500°C, but handling an easily ignited material at a high temperature is not preferable in an industrial process.

When the catalyst obtained as described above is used in the alkenylation reaction, various processes can be adopted for the reaction. Examples of such processes include a batch or semi-batch process in which raw materials are supplied into a reactor containing the catalyst batchwise or semi-batchwise, a complete mixed flow process in which the catalyst and raw materials are supplied continuously into a reactor, and a fixed bed flow process in which the catalyst is packed into a reactor and raw materials are passed through the catalyst. The process for the reaction should suitably be selected in accordance with the type of the reaction product which is the object of the process. In general, the selectivity of the monoalkenylaromatic hydrocarbon can be increased by adopting a process in which the aromatic hydrocarbon, one of the reactants, is present in an excess amount relative to the conjugated diene. For this purpose, a semi-batch process in which the conjugated diene is continuously supplied to the reactor semi-batchwise is preferable. When the reaction is conducted continuously in accordance with the complete mixed flow process or the fixed bed flow process, it is preferable that the concentration of the conjugated diene in the reactor can be decreased, for example, by separating the reactor into a plurality of stages and supplying the conjugated diene to individual stages because a higher selectivity can be obtained.

The conjugated diene having 4 or 5 carbon atoms which is used as another raw material in the alkenylation is used in such an amount that the ratio by mol of the conjugated diene to the aromatic hydrocarbon compound is generally in the range of 0.01 to 1, preferably 0.03 to 0.5. When the amount of the conjugated diene is more than the above range, further reaction of the formed monoalkenylaromatic hydrocarbon compound with the conjugated diene takes place, and the amount of compounds formed by addition of two or more molecules of the conjugated diene to one molecule of the aromatic hydrocarbon compound is increased. Polymerization of the conjugated diene also tends to take place. Therefore, the selectivity is decreased, and such an amount is not preferable.

The amount of the catalyst used in the alkenylation is generally in the range of 0.01 % by weight or more, preferably 0.05 % by weight or more, of the aromatic hydrocarbon compound used as the raw material.

The temperature of the reaction is generally in the range of 50 to 300°C, preferably 90 to 200°C. When the temperature is lower than the specified range, a sufficient rate of the reaction cannot be obtained although the reaction takes place, and the selectivity tends to be decreased. When the temperature is higher than the specified range, by-products such as tar are increased. Therefore, such temperatures are not preferable.

The pressure of the reaction is held at a pressure necessary for keeping the aromatic hydrocarbon compound used as the raw material and the products substantially in the liquid phase under the condition of the reaction. The pressure is generally in the range of 0.05 to 50 atm, preferably 0.1 to 20 atm, as the absolute pressure.

The reaction time of the alkenylation reaction, which is the reaction time in the batch process or the semi-batch process and the residence time in the complete mixed flow process, is generally 0.1 to 10 hours. In the fixed bed flow process, the LHSV (the liquid hour space velocity) of the aromatic hydrocarbon compound is generally 0.1 to 10 h⁻¹.

When the reaction is conducted in a suspension, the reaction fluid and the catalyst can be easily separated after the reaction by a conventional method, such as sedimentation, centrifugal separation, and filtration. The separated catalyst may be recycled into the reaction system or into the process for preparation of the catalyst after necessary treatments, such as removal of attached organic substances by burning in the air and washing of the catalyst with water.

### EXAMPLES

The present invention is described more specifically with reference to examples in the following. However, the present invention is not limited by the examples.

### Examples of Catalyst Preparation

### Catalyst A

To 100 ml of an aqueous solution containing 22.3 g of potassium hydroxide, 50 g of magnesium carbonate powder was added, and the mixture was stirred at 80°C for 1 hour. After the obtained mixture was dried at 140°C for one night, the dried mixture was calcined in the air at 500°C. The calcined mixture in an amount of 10 g was stirred in a nitrogen atmosphere at 200°C, and 1.0 g of sodium metal was added to the mixture. The obtained product was kept being stirred at the same temperature for 60 minutes to obtain catalyst A.

### Catalyst B

Catalyst B was obtained in accordance with the same procedures as those used for obtaining catalyst A except that 35 g of magnesium hydroxide powder was added in place of magnesium carbonate.

### Catalyst C

Catalyst C was obtained in accordance with the same procedures as those used for obtaining catalyst A except that 27.5 g of potassium carbonate was used in place of potassium hydroxide.

### Catalyst D

Catalyst D was obtained in accordance with the same procedures as those used for obtaining catalyst A except that 1.5 g of sodium metal was used.

### Catalyst E

Catalyst E was obtained in accordance with the same procedures as those used for obtaining catalyst A except that 10 g of the calcined mixture was stirred in a nitrogen atmosphere at 250°C, 1.0 g of sodium metal was added to the mixture, and then the obtained mixture was kept being stirred at the same temperature for 30 minutes.

### Catalyst F

Catalyst F was obtained in accordance with the same procedures as those used for obtaining catalyst A except that 50 g of calcium carbonate powder was used in place of magnesium carbonate.

### Catalyst G

Catalyst G was obtained by heating 10 g of potassium carbonate powder, which had been calcined at 500°C, in a nitrogen atmosphere at 200°C, adding 1.0 g of sodium metal to the obtained mixture while being stirred, and then heating the resultant mixture at the same temperature for 120 minutes.

### Example 1

To 10 g of catalyst A, 1,000 g of o-xylene which had been dewatered with molecular sieves was added under a nitrogen stream, and the obtained mixture was heated to 140°C. While the resultant mixture was stirred, 70 g of 1,3-butadiene was introduced in 1 hour to allow the reaction to proceed. After being cooled, the reaction mixture was deactivated by addition of isopropyl alcohol, and then a portion of the product was taken out and analyzed by the gas chromatography. The result of the reaction [the yield of 5-(o-tolyl)-2-pentene] is shown in Table 1.

### Examples 2 to 8 and Comparative Examples 1 and 2

The reaction was conducted in accordance with the same procedures as those conducted in Example 1 using various types and amounts of catalyst. In Comparative Example 2, 5.0 g of sodium metal was used as the catalyst. The results are shown in Table 1.

**Table 1**

| | type of catalyst | amount of catalyst (g) | yield (%) |
|---|---|---|---|
| Example 1 | catalyst A | 10 | 83.7 |
| Example 2 | catalyst A | 5 | 83.2 |
| Example 3 | catalyst B | 10 | 82.1 |
| Example 4 | catalyst B | 20 | 81.5 |
| Example 5 | catalyst C | 10 | 83.0 |
| Example 6 | catalyst D | 5 | 81.8 |
| Example 7 | catalyst E | 5 | 82.8 |
| Example 8 | catalyst F | 10 | 81.3 |
| Comparative Example 1 | catalyst G | 10 | 73.0 |
| Comparative Example 2 | Na metal | 5 | 5.3 |

### Example 9

To 10 g of catalyst A, 1,000 g of m-xylene which had been dewatered with molecular sieves was added under a nitrogen stream, and the obtained mixture was heated to 135°C. While the resultant mixture was stirred, 50 g of 1,3-butadiene was introduced in 1 hour to allow the reaction to proceed. After being cooled, the reaction mixture was deactivated by addition of isopropyl alcohol, and then a portion of the product was taken out and analyzed by the gas chromatography. The result is shown in Table 1.

### Examples 10 and 11

The reaction was conducted and the reaction product was examined in accordance with the same procedures as those conducted in Example 8 except that p-xylene or ethylbenzene was used in place of m-xylene. The results are shown in Table 2.

**Table 2**

| | aromatic compound used as raw material | product of the object | yield (%) |
|---|---|---|---|
| Example 9 | m-xylene | 5-(m-tolyl)-2-pentene | 86.5 |
| Example 10 | p-xylene | 5-(p-tolyl)-2-pentene | 85.9 |
| Example 11 | ethylbenzene | 5-phenyl-2-hexene | 82.1 |

As clearly shown in Examples, in accordance with the process of the present invention, industrially useful monoalkenylaromatic hydrocarbons, such as monoalkenylbenzenes, can be obtained from aromatic hydrocarbon compounds and conjugated dienes with high yields.

In the process of the present invention, the production can be conducted safely at a low cost because neither sodium metal nor potassium metal is used, and the process has a remarkable industrial advantage.

## Claims

1. A process for producing a monoalkenylaromatic hydrocarbon which comprises alkenylating a side chain of an aromatic hydrocarbon compound having at least one hydrogen atom at the α-position of the side chain with a conjugated diene having 4 or 5 carbon atoms in the presence of a catalyst comprising a solid material which has been obtained by calcining a mixture of a carbonate or a hydroxide of an alkaline earth metal and a potassium compound, adding an alkali metal to the obtained calcined product under an atmosphere of an inert gas, and subsequently heat treating the obtained mixture at a temperature which is the same as or higher than the melting point of the alkali metal.

2. A process according to Claim 1, wherein the temperature of the heat treatment is in the range of the melting point of the alkali metal to 500°C

3. A process according to Claim 1, wherein the alkaline earth metal is at least one type of metal selected from magnesium and calcium.

4. A process according to Claim 1, wherein the potassium compound is at least one type of compound selected from potassium hydroxide, potassium carbonate, and potassium hydrogencarbonate.

5. A process according to Claim 1, wherein the alkali metal is at least one type of metal selected from sodium and potassium.

6. A process according to Claim 1, wherein the aromatic hydrocarbon compound having at least one hydrogen atom at the α-position of a side chain is a monoalkylbenzene, a dialkylbenzene, a trialkylbenzene, a tetraalkylbenzene, a pentaalkylbenzene, a hexaalkylbenzene, a monoalkylnaphthanlene, a dialkylnaphthalene, a tetrahydronaphthalene, or indan.

7. A process according to Claim 1, wherein the conjugated diene having 4 or 5 carbon atoms is 1,3-butadiene, 1,3-pentadiene, or isoprene.
